# EUROPEAN PATENT APPLICATION

(11) **EP 3 878 405 A1**
(43) Date of publication of application: **15.09.2021**
(21) Application number: 19752567.8
(22) Date of filing: 28.06.2019
(51) Int. Cl.: A61F 2/08

(54) **MEDICAL DEVICE FOR TENSIONING OF LIGAMENTS ADJUSTABLE TO DIFFERENT ANATOMICAL LOCATIONS**

(30) Priority: 02.07.2018 PT 2018110815
(71) Applicant: Instituto Superior Técnico, 1049-001 Lisboa (PT)
(72) Inventor: MATOS, Henrique Homem De, 1495-094 ALGÉS (PT); COELHO, Carla Alexandra Madail, 1600-821 LISBOA (PT); VAZ, Maria De Fátima Reis, 2795-057 LINDA A VELHA (PT); REIS, Luís Filipe Galrão Dos, 1600-821 LISBOA (PT)
(74) Representative: Pereira da Cruz, Joao
(86) International application number: PCT/PT2019/050019
(87) International publication number: WO 2020/009597

(57) **Abstract**

The present invention refers to a medical device for tensioning grafts in orthopedic reconstruction of ligaments. The device comprises two subsets: the force applicator subset and the adapter subset. The first subset comprises two identical and symmetrical side arms and uses a helical spring system with a brake to quantify the tensile force. Each side arm comprises a rack (9) linked with a helical compression spring (4), a guiding axle (1), a suture wire securing part (8) and an outer chute (10) in which the rack (9) slides. In order to maintain the applied force, the brake uses a brake lever (12), an elastic pin (13) and a chute (10). The adapter subset has a "U" shape and comprises an alignment cone (17) which engages in the graft housing tunnel for controlling the direction of the traction force, and a fixation system composed of an adapter (16), by spikes (19) to be adjustable to the anatomical region and to promote a stable fixation.

## Description

### Field of invention

The present invention refers to a device used in orthopedic reconstruction of ligaments, which can be inserted in the field of medical devices, being part of a research and development division of biomechanical mechanisms.

The purpose of the present invention is to create a device which can be used in orthopedic reconstruction of ligaments, namely in the reconstruction of the anterior cruciate ligament of the knee, but which also adapts to other reconstruction surgeries of other ligaments in different anatomical regions, such as the elbow.

The device major function is to help the surgeon in the application of a force with a specific modulus and in a particular direction at the graft, during the phases of cycles tests carried out and at the fixation of the graft at the end of the surgery. This device has a quick, easily removable and maneuverable application system, which allows a stable fixation, when desired, and is little invasive in relation to the patient.

In this way, the present invention is quite advantageous in relation to the devices on the market, allowing control of the force modulus and its direction.

### State of the art

The close collaboration between Engineering and Medicine has allowed, in recent years, the development of devices and products that arise from the integrated combination of medical knowledge and new technologies. The present invention results from the combination of efforts between orthopaedic doctors and engineers.

With the adoption of a healthy lifestyle, there has been in recent years, an increase in the practice of sports. However, the increase in the number of sportive people leads to an increase in injuries. The most common injuries are the rupture of ligaments that can occur in the knees, hands, shoulders and elbows, being the most frequent a rupture of the anterior cruciate ligament (ACL) of the knee.

ACL reconstruction surgery is the sixth most frequent surgery in orthopaedics. Although there are several surgical techniques used to treat ACL ruptures, most orthopaedic doctors prefer the total reconstruction of the ligament, replacing the injured ligament with a graft. A graft is a tendon or ligament harvested from the patient himself or from a cadaver, which is prepared by the surgeon to be positioned in place of the damaged ligament. The preparation of the graft may include its folding, the ends being sutured, so that the graft is left with the ends attached to sutures. In surgery, holes are drilled near the patient's knee, through which the graft previously prepared is introduced, after removal of the injured ligament. In the final stage of the surgery, the surgeon needs to ensure that the graft is subjected to a predetermined tensile force, so that he has to apply a force that ensures its tensioning and to guide the direction of the force applied in the graft in the direction of the tibial tunnel axis. With this force applied, the surgeon performs several cyclic tests of the joint movement. If the medical requirements are met, the surgeon proceeds to the last stage of the surgery which involves graft fixation.

Many orthopaedic doctors choose to apply the force manually without knowing which force modulus is applied, avoiding the use of the devices on the market, due to the time spent during assembly and use of such devices.

In summary, this invention aims at the development of a medical device that offers the possibility of controlling the direction and the force modulus on the graft, in the tensioning phase during ligament reconstruction surgery.

According to the authors' research, the existing patents for such devices are as follows:
- Anterior cruciate ligament tensioning device and method for its use - US5713897 A.
- Graft ligament strand tensioner - US6547778 B1.
- Surgical tensioning assembly and methods of use - US 20110029079 A1.
- Suture separation and organization devices for use with graft tensioning device - US 7686810 B2.
- Method and apparatus for tibial fixation of an ACL graft - US 8771352 B2.

The devices on the market that present similar solutions are the TunneLoc Device (disposable) from Zimmer Biomet and the SE Graft Tensioning System (reusable) from ConMed.

The advantages of the present invention in relation to the existing devices on the market are: flexibility of use in different anatomical locations (knee, elbow, among others) through the adapter subset comprising the dowels (18), the spikes (19) and the adapter (16); quantification of the force applied to the graft during surgery by the helical compression springs (4) and the possibility of maintaining the force applied through the brake system composed by the brake lever (12) and the rack (9); alignment of the graft with the tibial tunnel by the alignment cone; and ease of handling of the device due to its simple structure and composed mainly of parts whose production can be performed by various manufacturing methods.

Given the characteristics of the invention, that is, being a surgical support device in the area of Health-Orthopaedics, the target market will be formed by hospitals, doctors and medical device companies.

### Summary of the invention

The present invention relates to a medical device for tensioning ligaments, adaptable to different anatomical locations. Orthopaedic surgeries of reconstruction of ligaments are very frequent and one of the problems of these surgeries is the lack of quantification of the force that is applied to the grafts in the final phase of the surgery, which might condition the graft performance. The surgeon applies, manually, a force to the graft without knowing its magnitude. One of the objectives of this device is precisely to quantify the applied force so that the procedure is repeatable. On the other hand, this device allows controlling the direction of the applied force.

The proposed device consists of two subassemblies: the force applicator subset responsible for the application of the force to the ligament, and the adapter subset that enables its application in different anatomical locations. The force applicator subset is formed by two identical and symmetrical lateral arms, a handle (14) and comprises as main elements, a helical compression spring (4) for the transmission of the traction force to the graft and a brake lever (12) to maintain the applied force. The adapter subset is comprised of a coupling system with coupling pins (21), a system of fixing dowels (18) and spikes (19).

The device in question is equipped with two side chutes (10), one on each side, which allow guiding the force to be applied, which will lead to tensioning of the graft, with two or four suture wires simultaneously. In each side chute there is a helical compression spring (4) which allows applying forces in the range of 5 to 60 N, whereby in the total of the two springs the force can vary between 10 and 120 N. The value of the predetermined force to be applied to the graft is read on the graduated scale of the guiding axle (1) placed inside the helical compression spring (4), which stays more exposed as the helical compression spring (4) is compressed.

The calibration mechanism is composed of a screw-nut(2), a guiding axle (1) with one of the extremities threaded, and a threaded calibration bushing (5) with a fine pitch thread that allows the calibration of the helical compression spring (4). The suture wires of the graft end are secured to the rollers of the suture wire securing parts (8). In turn, each suture wire securing part (8) is connected to a ball joint formed by a ball-receiving screw (6) and a ball-head screw (7), which makes possible to compensate for small differences in length which may exist between the suture wires and provides three rotational degrees of freedom. This system contributes to the standardization of the loading along the graft. When the user applies a force, the rack (9) coupled to the helical compression spring (4) through the threaded calibration bushing (5) moves from the proximal end of the suture wires, resulting in contraction of the helical compression spring (4).

The force applicator subset also includes a toothed unidirectional brake mechanism which blocks the movement of the helical compression spring (4), preventing the variation of the force. The brake mechanism is composed of a rotatable brake lever (12) which allows the rack (9) to be displaced in one direction only, during compression of the helical compression spring (4). The geometry of the brake lever (12) with a tooth has been designed to engage into the teeth of the rack (9) preventing it from moving contrary to the compression direction, acting as a brake. At the other extremity of the brake lever (12) is fixed a micro-spring (11) which enables the brake lever (12) to return to the resting position. This movement may be counteracted, for example, when we want to deactivated the brake, with the application of a force at the same end, whenever the user demands. The centre of rotation of the brake lever (12) and the connection between the chute (10) and the brake lever (12) are ensured by an elastic pin (13).

The handle mechanism has been designed taking into account the possibility of rotation of the handle (14), with an ergonomic geometry that provides greater comfort to the user. The mechanism of rotation of the handle and the distance between the two arms, where the helical compression springs (4) work, allow the manipulation of the auxiliary tools during surgery.

The adapter subset comprises the adapter (16), the connecting pieces (15), the cylindrical pins (20) and the coupling pins (21) and has been designed to be compatible with the different reconstruction techniques and to be adaptable to different anatomical locations. The assembly method is simple and fast. The coupling between the adapter subset and the force applicator subset is performed by the coupling pins (21) and spherical end spring screws (23). One of the extremities of the coupling pin (21) is fixed in the connecting piece (15) of the adapter subset, the other end of the coupling pin (21), which receives the chute (10), has a groove for receiving the spherical tip of the spherical end spring screw (23). The spherical end spring screw (23) secures the chute (10) and the coupling pin (21), defining the force required for the decoupling.

The adapter subset has the possibility of having two fastening mechanisms: fixing with four dowels (18) and fixing with two spikes (19). Thus, the adapter (16) has four through holes where the dowels (18) can be engaged and there are two blind holes where two spikes (19) are fitted. The four through holes allow various combinations of use of the dowels (18) so that the device easily adapts to different anatomical locations, without requiring the user to apply a force, which is a rather stable solution.

The second method of assembly is made through two small spikes (19) and an elastic strap (optional) which prevent the movement of the adapter (16). In these cases, the user does not want a fixation with high stability, due to anatomical or clinical reasons. The fixation with the dowels (18) is used when a more stable fixation is required, as opposed to the fixation with the use of only the spikes (19) in which there is only an accommodation or contact of the device to the patient.

### Detailed description of the invention

Figure 1 shows the device in exploded view, identifying the constituents of the force applicator subset and the adapter subset.

Figure 2 presents the two subsets of the device, the force applicator subset and the adapter subset.

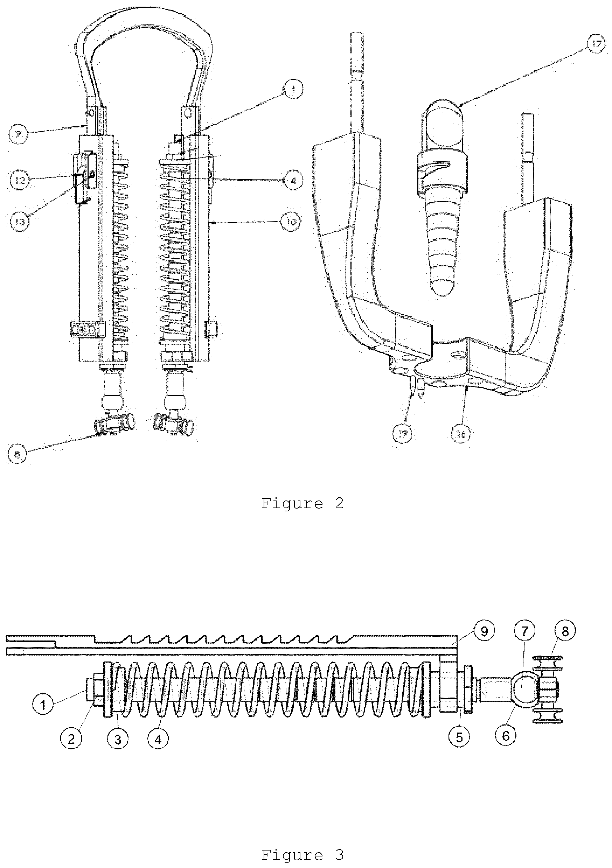

In Figure 3 one of the side arms of the device is shown, being the other arm symmetrical to this one, and the helical compression spring (4) mechanism of the force applicator subset. A guiding axle (1) is placed inside the helical compression spring (4). At one end where the axle is threaded, a screw-nut (2) and a bushing (3) contact the helical compression spring (4). At the other end, the axle is connected to a threaded calibration bushing (5), which functions integrally with the ball-receiving screw (6) and the ball-head screw (7). When a force is applied by the user, the threaded calibration bushing (5) slides on the guiding axle (1), compressing the helical compression spring (4) against the extremity where the screw-nut (2) stands. The helical compression spring (4) is confined between the screw-nut (2) and the threaded calibration bushing (5), which in turn is limited by a retaining ring (24). The suture wire securing part (8) functions integrally with the guiding axle (1). The suture wire securing part (8) has two rollers where the user clamps the wire sutures manually.

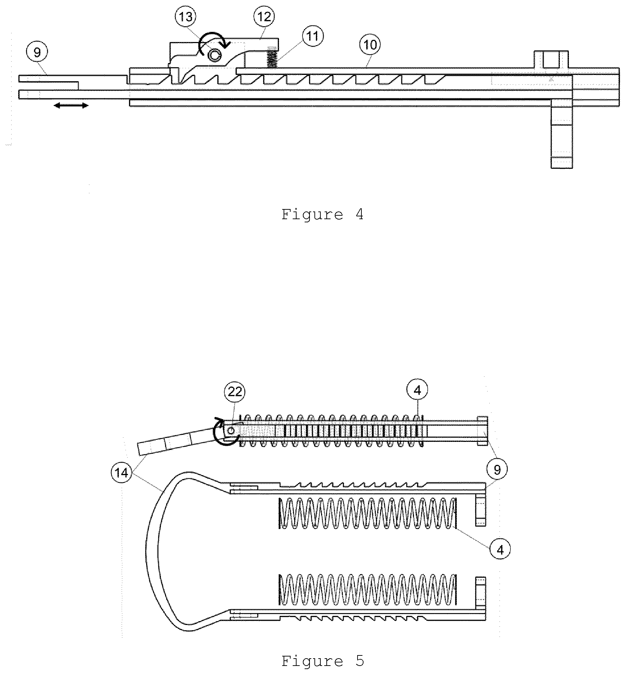

In each of the side arms of the device, the spring mechanism is connected through a sawed or toothed piece also called a rack (9) to the braking mechanism (Figure 4). The brake is constituted by the rack (9), the brake lever (12), an elastic pin (13), the micro-spring (11) and the chute (10). The brake lever (12) has two positions, one of which allows the rack (9) to move and the other prevents its movement by engaging one of the teeth. The brake lever (12) has at one end a tooth for engaging into the rack (9) and at the other end a micro-spring (11) is positioned which allows the brake lever (12) to return to the resting position.

The elastic pin (13) acts as the center of rotation of the brake lever (12) and allows the connection between the rack (9) and the chute (10).

Figure 5 shows the handle (14) rotation mechanism. The handle (14) is attached to the rack (9) through a round head pin (22) .

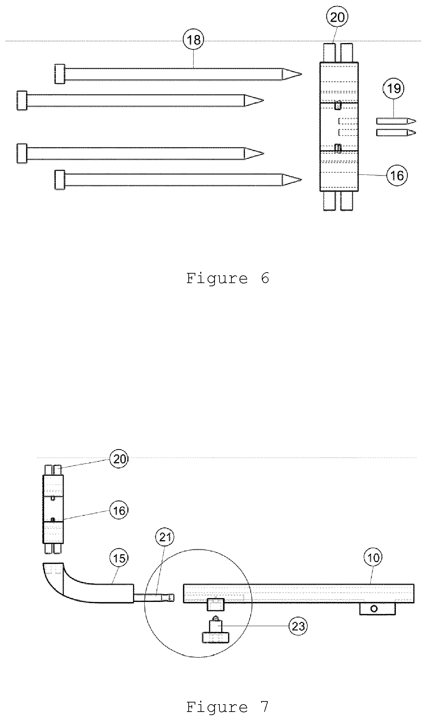

In Figure 6 the adapter subset is schematized. The adapter (16) has four through holes, which allow the passage of four dowels (18) of variable length, which is predefined by the user. The adapter (16) also has two blind holes where the spikes (19) are mounted.

Figure 7 presents the scheme of the connection of the adapter subset to the force applicator subset through the connecting piece (15), and the coupling pin (21) in the chute (10) of the force applicator subset, both by fitting. In order to sustain the relative movement between the force applicator subset and the adapter subset, a spherical end spring screw (23) is placed which immobilizes the coupling pin (21) within the chute (10).

### Description of the figures

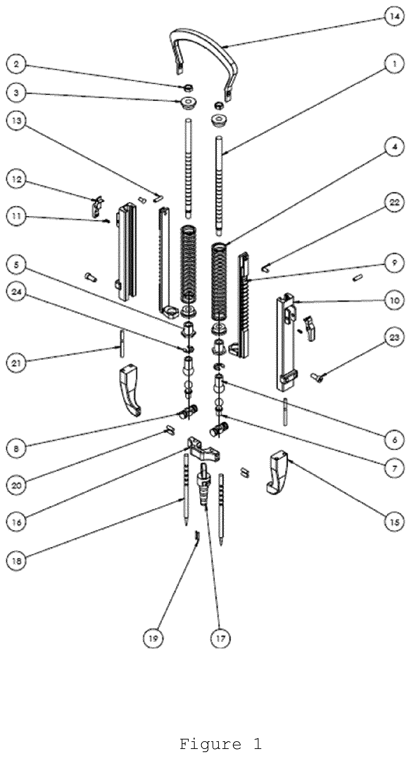

Figure 1 shows the entire device in exploded view. In the force applicator subset, the following components are shown: guiding axle (1); screw-nut (2); bushing (3); helical compression spring (4); threaded calibration bushing (5); (6) ball-receiving screw (6); ball-head screw (7); suture wire securing part (8); rack (9); chute (10); micro-spring (11); brake lever (12); elastic pin (13); handle (14); round head pin (22); spherical end spring screw (23) and retaining ring (24). In the adapter subset, the following components are shown: connecting piece (15); adapter (16); alignment cone (17); dowels (18); spikes (19); cylindrical pins (20) and coupling pins (21).
Figure 2 shows the two subsets of the device, the force applicator subset and the adapter subset. The first subset comprises two identical, symmetrical side arms and uses a spring brake system to quantify the tensile force modulus. Each side arm comprises a rack (9) linked with a helical compression spring (4), a guiding axle (1), a suture wires securing part (8) and an outer chute (10) in which the rack (9) slides. In order to maintain the applied force, the brake uses the brake lever (12), an elastic pin (13) and a chute (10). The U-shaped adapter subset comprises an alignment cone (17) which engages in the graft housing tunnel for controlling the direction of the tensile force, and a fixation system composed of an adapter (16), by dowels and spikes (19) to adapt to the anatomical region and to promote a stable fixation during the stage of cyclic tests and at the final stage of graft fixation.
Figure 3 presents the spring mechanism as well as the suture securing mechanism comprising a guiding axle (1), a screw-nut (2) and a bushing (3) of the helical compression spring (4). The other extremity of the guiding axle (1) is supported by a threaded calibration bushing (5) which is connected to a ball-receiving screw (6) and a ball-head screw (7). There are also shown in figure 3 the suture wires securing part (8) and the rack (9).
Figure 4 displays the brake mechanism formed by a toothed or sawed piece, also referred as a rack (9), a chute (10), a micro-spring (11), a brake lever (12) and an elastic pin (13) .
Figure 5 exhibits the mechanism of rotation of the handle (14). The handle (14) is connected to the rack (9) through a round head pin (22). It is possible to observe the distance between the helical compression springs (4).
Figure 6 shows the adapter (16) having four through holes, which allow to pass four dowels (18) of variable length, which is predefined by the user. The adapter has two other blind holes where the spikes (19) are mounted. Also shown in Figure 6 is the connection between the adapter (16) and the connecting piece by cylindrical pins (20).
Figure 7 schematically illustrates the connection of the adapter subset to the force applicator subset through the coupling pin (21) in the chute (10) of the force applicator subset, both by fitting. In order to sustain the relative movement between the force applicator subset and the adapter subset a spherical end spring screw (23) is placed. Figure 7 also shows the connection between the adapter (16) and the connecting piece (15) by cylindrical pins (20).

### Example

During the graft tensioning stage and in the fixation phase, the adapter subset is fitted to the patient's leg near the tibial tunnel opening before the graft is inserted into the knee. The alignment cone (17) is inserted into the tunnel until it is fitted. Due to its variable diameter geometry this adjustment is almost automatic.

Then the user has two possibilities for fixing the adapter subset: the fixing by dowels (18) and the fixing with two spikes (19). The first fixing method is carried out using dowels (18) of varying length, which pass inside the through holes in the adapter (16) embedded in the bone. The through holes allow various combinations of use of the dowels (18) so that the device easily adapts to different anatomical regions. The second fixation method is accomplished by two spikes (19) and an elastic strap (optional), fitted into the blind holes of the adapter (16), which upon contact with the bone prevent the movement of the adapter subset. In these cases, the user does not want a fixation with great stability, for anatomical or clinical reasons. The fixation with the dowels (18) is used when a more stable fixation is required, in opposition to the fixation with the use of only the spikes (19) in which there is only an accommodation or contact of the device to the patient.

After the stability of the fixation is ensured, the alignment cone (17) is removed with a slight rotation and the suture wires of the graft are placed in the empty space left by the alignment cone (17). Then the force applicator subset is attached to the adapter subset using the coupling pins (21). The user may control the required force through the spherical end spring screw (23) to ensure that the subassemblies do not separate at the stage of tensioning.

The device is installed after insertion of the sutures wires into the suture wire securing parts (8).

To apply the tensile force to the graft, the user must place one hand on the handle (14) and the other hand on the lateral chute (10) of the device, so that the user is comfortable to perform the traction movement. If the user moves the handle (14) away from the knee, the graft begins to be tensioned. The modulus of the applied force can be observed on the scale, as the inner guiding axle (1) of the helical compression spring (4) is exposed.

Upon reaching the required tensile force, the user can release the handle (14), and the brake lever (12) is forced to return to the resting position by the micro-spring (11). After performing the cyclic tests for graft adjustment in the housing tunnels, the user can change the force applied to the graft, if it is necessary. Once the user is satisfied with the applied force to the graft tensioning, the user can fix the graft to the tibia. At this stage the dowels (18) are removed and the device is taken out from the leg. At the end of the surgery the device is sterilized in autoclave along with the other tools used.

## Claims

1. Medical device for tensioning of ligaments, adjustable to different anatomical locations, **characterized in that** it comprises:
a) a force applicator subset, with two side arms, consisting of the following components:
i. guiding axle (1);
ii. screw-nut (2);
iii. bushing (3);
iv. helical compression spring (4);
v. threaded calibration bushing (5);
vi. ball-receiving screw (6);
vii. ball-head screw (7);
viii. suture wire securing part (8);
ix. rack (9);
x. chute (10);
xi. micro-spring (11);
xii. brake lever (12);
xiii. elastic pin (13);
xiv. handle (14);
xv. round head pin (22);
xvi. spherical end spring screw (23);
xvii. retaining ring (24);
b) an adapter subset with a "U" shape, consisting of the following components:
xviii. connecting piece (15);
xix. adapter (16);
xx. alignment cone (17);
xxi. dowels (18);
xxii. spikes (19);
xxiii. cylindrical pins (20);
xxiv. coupling pins (21).

2. Medical device according to claim 1, **characterized by** comprising a spring system in each arm of the force applicator subset, being the helical compression spring (4) connected to the guiding axle (1) by the bushing(3), and connected to the rack (9) by the threaded calibration bushing (5).

3. Medical device according to claim 1, **characterized by** comprising a toothed unidirectional brake mechanism composed by a rack (9) and a brake lever (12) connected to the chute (10) by the elastic pin (13).

4. Medical device according to the preceding claims, **characterized by** comprising a force applicator subset containing a scale on the guiding axle (1) for reading the value of the applied force to the graft.

5. Medical device according to claim 1, **characterized by** comprising a suture wire securing part (8), composed of a suture wire securing part (8), connected to the ball-head screw (7), which in turn attaches to the ball-receiving screw (6).

6. Medical device according to claim 1, **characterized in that** the adapter subset comprises a coupling system to the force applicator subset, composed of the coupling pins (21) that connect the connecting piece (15) to the chute (10), and by the spherical spring screws in contact with the coupling pins (21).

7. Medical device according to the preceding claims, **characterized in that** the adapter (16) has a variable anatomical configuration according to the anatomical area of application.

8. Medical device according to the preceding claims, **characterized in that** the adapter (16) is connected to the connecting piece (15) through the cylindrical pins (20) .

9. Medical device according to the preceding claims, **characterized by** comprising a fixing system with two fixing options.

10. Medical device according to claims 1 and 9, **characterized in that** the fixing system is composed of dowels (18) passing through the holes of the adapter (16) .

11. Medical device according to claims 1 and 9, wherein the fixing system is composed of spikes (19) fitted in the adapter (16).

12. Medical device according to claims 1, 9 and 11, **characterized by** having an elastic strap.

13. Medical device according to the preceding claims, **characterized by** having an alignment cone (17) fitted in the graft housing tunnel.

14. Medical device according to the preceding claims, **characterized by** having an alignment cone (17) connected to the adapter (16).

15. The use of the medical device defined in claims 1 to 14, **characterized by** comprising the following steps:
a) The adapter subset is settled on the surface of the required anatomical region, by leaning the ends of the spikes (19) to the bone and inserting the alignment cone (17) into the graft housing tunnel;
b) The adapter subset is secured to the anatomical region using one of the fixing options defined in claims 9, 10, 11 and 12, using dowels (18) or spikes (19);
c) The alignment cone (17) is removed from the adapter (16) through a rotational movement;
d) The force applicator subset is coupled to the adapter subset, fitting the chutes (10) in the coupling pins (21);
e) The spherical spring screws are fastened with the coupling between the two subsets;
f) The suture wires of the graft are placed in the suture wire securing parts (8);
g) Graft tensioning is performed by applying a force on the handle (14) which in turn is transmitted to the graft through the helical compression springs (4) ;
h) The value of the tensile force is read on the scale of the guiding axle (1);
i) The brake mechanism is powered by the micro-spring (11) which causes a rotation of the brake lever (12) that gets in contact with the rack (9);
j) The handle (14) rotates about the round head pin (22) to give passage to the surgical auxiliary tools;
k) After fixation of the graft to the bone, the medical device is removed from the anatomical region.
